# EUROPÄISCHE PATENTSCHRIFT

(11) **EP 2 554 192 B1**
(45) Veröffentlichungstag und Bekanntmachung des Hinweises auf die Patenterteilung: **14.05.2014**
(21) Anmeldenummer: 12001826.2
(22) Anmeldetag: 17.03.2012
(51) Int. Cl.: A61M 1/16

(54) **Kombination eines Einzelplatz-RO-Gerätes mit einem Hämodialysegerät**
Combination of a single point RO device with a haemo-dialysis device
Combinaison entre un appareil d'osmose inverse monoposte et un appareil d'hémodialyse

(30) Priorität: 01.08.2011 DE 102011109093
(43) Veröffentlichungstag der Anmeldung: 06.02.2013
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- WO-A2-2009/036717
- DE-A1-102009 057 562
- US-A- 5 032 265
- US-A1- 2010 326 916

## Beschreibung

Die Erfindung betrifft die Kombination eines Einzelplatz-Umkehrosmosegeräte (RO-Gerät) mit einem Hämodialysegerät (HD-Gerät), wobei das RO-Gerät einen Filter mit einer Membran aufweist, die einen Primärraum von einem Sekundärraum trennt, wobei in den Primärraum eine Rohwasserzufuhrleitung einmündet und der Sekundärraum über eine eine Leitfähigkeits-Messeinrichtung enthaltende Verbindungsleitung mit einem Wassereingangsventil des HD-Gerätes verbunden ist, und wobei der Konzentratausgang des Primärraums über eine einen Strömungswiderstand enthaltende Konzentratleitung zu einem Abfluss führt, wobei das Permeat bei Anforderung durch das HD-Gerät entweder über das Wassereingangsventil in das HD-Gerät oder über ein Spülventil des HD-Gerätes zu einem Abfluss fließt.

Ziel dieser Entwicklung ist es, Verbraucher, insbesondere Hämodialysegeräte, mit einem möglichst geringen Installationsaufwand kostengünstig mit chemisch und hygienisch hochwertigem, hochreinem Permeat zu versorgen.

Weitere Anwendungen dieser Entwicklung für andere Bereiche wie z.B. für Labor-, oder Biologie-, oder auch Pharmazietechnologie, als Gerät für die Produktion hochreiner Spülflüssigkeit, oder auch zur Produktion von Flüssigkeit zur Herstellung von Medikamenten, Zellkulturen und dergleichen sind vorstellbar und praktikabel.

Insbesondere im Bereich der Hämodialyse werden in der Regel Zentralversorgungs-Umkehrosmoseanlagen mit entsprechend aufwändig zu installierenden Permeatversorgungsleitungen eingesetzt. Gravierende Nachteile der zentralen Umkehrosmoseanlagen sind zum einen das hohe Risiko einer fehlenden Behandlung bei Geräteausfall, zum anderen die hohen Installationskosten und die problematische Hygienesicherheit der Installation.

Einzelplatz Umkehrosmosen werden hauptsächlich aus räumlichen Gründen im Intensivmedizinischen Bereichen verwendet. Für die chronische Hämodialyse HD ist die Versorgung der Dialysegeräte mit Permeat durch Einzelplatz RO Anlagen zur Zeit aus Kostengründen nicht praktikabel.
Weitere Schwierigkeiten sind bei der Kombination von RO- Anlage und HD Gerät der fehlende Nachweis, dass die Permeatversorgung aus Gründen des Keimeintrages in das HD Gerät totraumfrei und komplett desinfizierbar erfolgt.

Dazu wird gemäß dem Stand der Technik eine integrierte chemische oder thermische Desinfektion des Verteilungssystems inkl. der HD Geräte durchgeführt.

Ein anderer gravierender Mangel ist die sinkende Effizienz und Lebensdauer der Umkehrosmosemembran in Folge von nicht reversiblen Ablagerungen, da sich im Versorgungswasser für die RO- Anlage häufig Biomasse und schwerlöslichen Salze befinden.

Die DE 10 2009 057 562 A1 offenbart eine Umkehrosmose-Anlage mit Verteilungssystem für Permeat mit den Merkmalen des Oberbegriffs des Patentanspruchs 1. Die Anlage enthält eine UV-Lampe in dem Wassereingangsbehälter und eine elektrolytische Ozonzelle in einer Permeatleitung. Die WO 2009/036717 A2 offenbart ein Filtermodul, das ein Permeatsammelrohr aufweist.

Zweck und Ziel der Erfindung ist deshalb, die Permeatversorgung eines HD-Gerätes mit minimalem technischem Aufwand, bei gleichbleibend hoher Membranleistung und bester mikrobiologische Qualität zu gewährleisten.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Diese Aufgabe wird wirkungsvoll dadurch gelöst, dass die RO-Anlage bevorzugt eine Disposable d.h. Wegwerfmembran beinhaltet und nur mit einem minimalen technischen Aufwand so ausgestattet ist, dass erst bei einer Kopplung mit dem HD Gerät die volle Funktion so zur Geltung kommt, dass zum einen durch die gemeinsame Nutzung von Funktionselementen die Permeatzuführung chemisch einwandfrei und totraumfrei erfolgt und zum anderen durch die Kommunikation zwischen HD-Gerät und RO-Anlage ein Ressourcen sparender Einsatz an Wasser und Energie möglich ist.

Mit großem Vorteil werden dabei Ventile des HD Gerätes zur Freigabe oder Spülung der hochreinen Verbindungsleitung verwendet.
Dabei nimmt die präventive Desinfektion der hochreinen Verbindungsleitung zum HD Gerät eine herausragende Stellung ein. Dazu sieht die Erfindung mit Vorteil eine Reinigungskammer im Permeatsammelrohr der Umkehrosmosemembran vor. Vorteilhaft wird auch auf der Primärseite der Umkehrosmose eine Reinigungskammer vorgesehen.
Die Aufgabe der Reinigungskammern ist zum einen die Dekontamination der Mikroorganismen u. zum anderen die Stabilisierung der Härtebildner, so dass effizienzmindernde Ablagerungen auf der Umkehrosmosemembran verhindert werden.

Dies wird durch die Konstruktion der Reinigungskammer, die eine elektrische oder magnetische oder elektromagnetische oder elektrolytische oder sonographische oder eine Kombination verschiedener physikalischer Einwirkungen der durchfließenden Flüssigkeit zulässt, bewirkt. Experimentell wurde auch nachgewiesen, dass mittels einer Wasserbestrahlungsfrequenz im UKW Frequenzbereich von vorzugsweise 13,56 MHz eine Elektrolyse stattfindet. Auch dieses Form der Reinigungskammer ist als Dekontaminations- Vorrichtung einsetzbar.

Mikroorganismen werden dabei entweder oxidiert oder durch elektrische Impulse an der Vermehrung gehindert bzw. reduziert.

Die physikalische Kalkschutzfunktion besteht in der Stabilisierung des im Wasser gelösten Kalkes so, dass die normalerweise großen Wassermolekülcluster mit Ihrer-dipolartigen- elektrischen Ladung aufgebrochen werden und sich so ordnen, dass vorwiegend kleinste Wassermoleküle Cluster entstehen, die nicht oder nur im geringen Umfang zu Ausfällen neigen.

Die Anwendung und der Einbauort der Reinigungskammern sind jedoch nicht auf die beschriebene Funktion beschränkt.

Da die Desinfektionswirkung der elektrolytisch erzeugten Sauerstoffradikalen als auch die Stabilisierung der Kalkkristalle in der Flüssigkeit nach dem Abschalten der Reinigungskammer nur temporär vorhanden sind, wird vorteilhaft periodisch und oder am Ende eines Betriebszyklus der Strömungswiderstand der RO-Anlage, mittels Bypassventil geöffnet. Dadurch wird die Strömung im Primärzirkulationskreis schlagartig erhöht und die Oberflächen der Flüssigkeit führenden Komponenten schwallartig überströmt und ausgespült

Zur Unterstützung der desinfizierenden Wirkung kann eine Beschichtung des Membransammelrohres oder der Membran- und Spacermaterialien mit antimikriobioziden Mitteln erfolgen.
Da die Wirkung der Reinigungskammer durch ihren physikalischen Effekt oder ihre Effekte auf die Kristallbildung oder Verkeimung durch den Anwender nicht unmittelbar feststellbar ist, kann mit Vorteil für Primär- wie Sekundärraum ein Reinigungssensor vorgesehen werden.

Dabei können Komponenten oder flüssigkeitsführende Leitungen mit transparentem oder transluzentem Material ausgestaltet sein, um visuell oder opto- elektronisch die Verunreinigung zu überprüfen.

Bei einer vorteilhaften Ausgestaltung ist die Sender-Empfängereinheit auf einer Ebene angeordnet. Das optische Sendersignal wird dabei auf eine gegenüberliegende Spiegelfläche projiziert und von dort zum opt. Empfänger zurückgestrahlt.

Eine weitere Ausgestaltung des Verunreinigungssensors besteht darin, dass der Sensor die Ablagerung von biologischen Schmutzschichten bestimmt, indem diese Ablagerung durch Anstrahlung z.B. mit UV-Licht ein der Schichtdicke entsprechendes fluoreszierendes, messbares Antwortsignal reflektiert.

Mit großem Vorteil kann zur Verbesserung der zeitlichen Einwirkung und Verstärkung der physikalischen Reinigungseffekte eine zusätzliche Zirkulationspumpe mit einer Reinigungskammer zwischen den Konzentratausgang und dem Mischwassereingang angeschlossen werden. Dabei kann es sich um eine zusätzliche Reinigungskammer mit einem differenten physikalischen Effekt zur Reinigungskammer handeln.

Die Durchströmung des Primärraumes im Sinne einer optimalen Überströmung der Membran ist dabei gewährleistet, und zwar weitgehend unabhängig von der Tätigkeit der für die Mischwasser-Zuführung, den Druckaufbau und Zirkulationsleistung eingesetzten Pumpe

Durch diese Erfindung kann sowohl eine geregelte präventive Vermeidung des Biofilmes im Primärkreis der Membran, als auch eine kostensparende totraumfreie Desinfektion der Permeatzuführung erfolgen.

Es ist vorstellbar, dass die in dieser Erfindung benannte Umkehrosomose Membran auf Grund der rasanten Entwicklung selektiver Hohlfasermembranen schon in Kürze durch eine dieser Erfindung ähnliche Kombination von HD Gerät und vorgeschalteter - günstiger zu produzierender- Hohlfaser - Enthärtungs-/Sterilfiltermembran ersetzt wird.

Das Funktionsprinzip von Umkehrosmoseanlagen besteht bekanntlich darin, dass das aufzubereitende Wasser in einem Filtermodul unter Druck an der Oberfläche einer semipermeablen Membran entlanggeführt wird, wobei ein Teil des Wassers, das sogenannte Permeat, durch die Membran tritt und auf der anderen Seite der Membran gesammelt und den Verbrauchsstellen zugeführt wird. Der nicht durch die Membran tretende, mit zurückgehaltenen Stoffen angereicherte Teil des Rohwassers, das sogenannte Konzentrat, fließt am Ende der Strömungsstrecke des Primärraumes aus dem Membranmodul aus.

Bei selektiven Hohlfaser-Membranen handelt es sich ebenso um ein Filtrationsverfahren bei dem bestimmte Wasserinhaltstoffe zurückbehalten werden, um eine für die Hämodialysebehandlung brauchbare Flüssigkeit zu filtrieren. In diesem Fall ist die Anwendung oxidierender Desinfektionsmittel und der Einsatz von Reinigungszellen auf der Sekundärseite bzgl. Materialverträglichkeit und Konstruktion geringfügig anzupassen.

Der nachfolgende Text beschreibt den Einsatz der RO Membran.

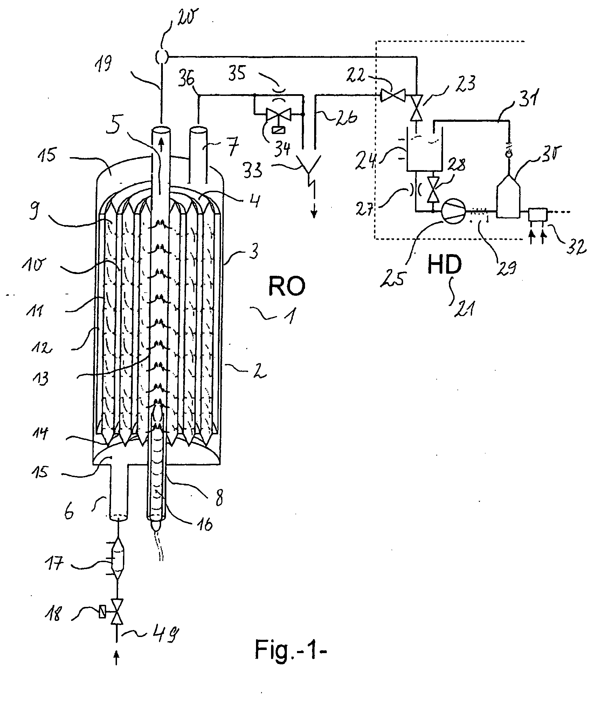

Das Schema in Figur 1 stellt die Kombination einer Umkehrosmose (RO) 1 mit einem Hämodialysegerät (HD-Gerät) 21 und dem Zusammenwirken gemeinsamer Funktionselemente dar.

Das aufzubereitende Rohwasser fließt aus der zuführenden Leitung 49 über das Ventil 18 durch eine Reinigungskammer 17 in das Druckrohr 3, das mit einer RO Membran 4 bestückt ist. Der Primärraum der RO-Membran bzw. der Wasserzuführkanäle 10 ist durch die semipermeable Membran 11 von dem Sekundärraum 9 bzw. Permeattaschen getrennt.

Aus den Permeattaschen 9 fließt das Permeat durch die Permeatsammelrohrperforierungen 13 über das Permeatsammelrohr 5 und Verbindungsleitung 19 zu dem HD Wassereingangsventil 23 des HD Gerätes 21.

Überschüssig erzeugtes oder durch die Leitfähigkeitszelle 20 gemessenes fehlerhaftes Permeat kann am Ende der Leitung 19 über ein eingefügtes HD Spülventil 22 mit Druckhaltefunktion zum Abfluss 33 fließen.

Der für die Filtration notwendige Druck im Primärraum des RO-Filters 2 wird mit einem in die Konzentratleitung 36 stromabwärts vom RO Filter eingefügten Strömungswiderstand 35, z.B. in Form eines Drossel- oder eines Druckhalteventils, erzeugt.

Bei Anforderung von Permeat durch das HD-Gerät (21) öffnet das Wassereingangsventil (18), und nach Freigabe durch die LF-Zelle 20 wird das HD Gerät über das HD Wassereingangsventil 23 versorgt.

Bei nicht ordnungsgemäßer Permeatqualität oder auch bei notwendigen Spülprogrammen wird HD Wassereingangsventil (23) geschlossen und Spülventil (22) des HD-Gerätes geöffnet, so dass das nicht genutzte Permeat oder die Spülflüssigkeit über die Spülleitung (26) zum Abfluss (33) fließt.

Im HD Gerät 21 wird das eingebrachte Permeat über die Entgasungsdrossel (27), die Pumpe (25) des HD-Gerätes, die Heizung (29) und die Entgasungskammer (30) für die HD-Behandlung aufbereitet. Das HD Gerät beinhaltet eine Zirkulationsleitung (31) und eine Konzentrat- und Bicarbonatzuführung (32).

Das Permeatsammelrohr (5) besitzt eine Aufnahme (8) für eine Reinigungszelle (16), deren Elektroden (40) Oxidationsmittel, wie z.B. atomaren und oder elementaren Sauerstoff oder Ozon oder OH Hydroxile oder Funkwellen zu Erzeugung von Oxidationsmitteln in das Permeat abgeben.

Das erzeugte Oxidationsmittel wird mit dem Permeat durch die Leitung (19) und das zunächst geöffnete Spülventil (22) zum Abfluss (33) geführt.

Zur Reduzierung des Spülflusses kann dabei das Ventil (22) getaktet werden. Es ist auch die Hinzunahme eines zusätzlichen nicht aufgezeigten Strömungswiderstandes möglich.

Nicht dargestellt ist eine Verunreinigungszelle zum Detektieren organischer oder anorganischer Ablagerungen, sowohl innerhalb der Konzentratleitung (36) als auch in der Permeatleitung (19).

Um Ablagerungen auf der Primärseite der Membran, d.h. auf der Innenseite des Druckrohres (3), den Flüssigkeitskanälen (10) und Abflussleitung (36)] zu vermeiden, kann sowohl während des Spül- als auch Versorgungsvorgangs für das HD-Gerät 21 die Reinigungszelle (17) mit eingeschaltet werden.

Vorzugsweise wird in zyklischen Spülabständen das Spülventil (34) geöffnet und der gesamte Primärraum 10 durchströmt und ausgespült.

Die dargestellten Aktoren und Sensoren können dabei sowohl durch das HD-Gerät oder auch in Kombination von HD-Gerät und RO gesteuert werden. Ebenso ist die räumliche Anordnung dieser Funktionselemente auch als Bestandteil des HD-Gerätes möglich.

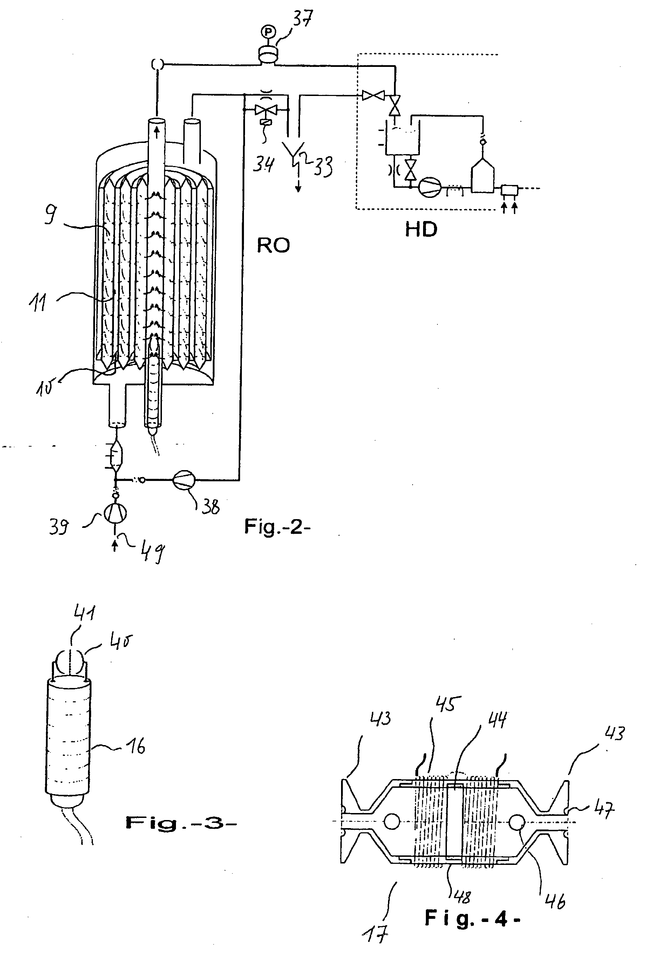

Figur 2 zeigt ergänzend eine Pufferkammer (37) die zum einen der schnelleren Permeatlieferung in das HD-Gerät (21) und zum anderen zur Erzeugung eines negativen Transmembrandruckes dient. Bei Erzeugung eines negativen Transmembrandruckes wird die Filtrierrichtung durch Unterbrechen der Wasserzufuhr (49) und Öffnen des Spülventils (34) umgekehrt. Dabei fließt das im Puffergefäß (37) befindliche Permeat über die Permeattaschen (9) zurück zur Primärseite (10) und löst dabei die auf der Membranoberfläche (11) befindlichen Ablagerungen. Mit Öffnen der Wasserzufuhr (49) wird diese zum Abfluss (33) abgeschwemmt.

Pumpe (39) erhöht den Druck im Primärraum und verbessert damit die Filtrierleistung.
Die Zirkulationspumpe (38) trägt ebenfalls zur Leistungserhöhung, insbesondere zur Wassereinsparung bei, indem die Überströmung auf der Primärseite einen größeren Anteil im Verhältnis zur Permeatleistung erhält.

Figur 3 zeigt schematisch eine Reinigungszelle mit 2 Elektroden 40, die als Anode, Kathode und Kationentauschermembran als Elektrolysezelle formschlüssig, dichtend in das Permeatsammelrohr eingebracht werden kann.

Figur 4 zeigt beispielhaft den Aufbau einer Reinigungszelle (17) mit 3 Elektroden, wobei die mittlere Elektrode (44) räumlich und elektrisch von den beiden Außenelektroden (43) isoliert ist.

Durch Materialauswahl und elektrische Anschlussart ist es möglich die Reinigungskammer (17) als Elektrolysezelle oder als elektromagnetische Zelle oder als Zelle mit Elektrodenanschlüssen für Strom und Spannung - auch kapazitiv - zu betreiben.
Dabei wird vorzugsweise ein Pol der elektrischen Versorgung an die gebrückten Außenelektroden (43) - und der andere Pol an die mittlere Elektrode (44) angelegt.

Bei Betrieb der Reinigungskammer (17) als Elektrolysezelle sind die beiden Außenelektroden (43) die Kathoden, die mittlere Elektrode (44) die Anode. Bei der Anwendung als Elektrolysezelle ist es zur Erzielung eines höheren Wirkungsgrades vorteilhaft Anoden und Kathodenraum durch Kationentauschermembranen zu trennen. Aufgrund der geringen Permeabilität dieser Membran ist die Anordnung von Anode, Membran, Kathode in eine nicht dargestellte strömungstechnisch günstige Ausführung zu ändern.
Diese Elektrolysezelle dient der Herstellung von Sauerstoffradikalen für die Inaktivierung der Mikroorganismen oder auch zur Reduzierung der Kalkablagerung.
Figur 4 zeigt den Aufbau einer kombinierten Reinigungskammer (17) mit 3 Elektroden und einer Spulenwicklung (45).
Hierbei wird die Entkalkung über die Kraftlinien des von der Spule erzeugten Magnetfeldes in der Flüssigkeit durchgeführt.
Die Verwendung von teflongekapselten Ringmagneten in der Flüssigkeit oder Ringmagneten außerhalb des Isolierstückes (48) anstelle der Spulenwicklung (45) ist möglich.

Zusätzlich zu den gezeigten Darstellungen sind diverse Vorfiltrations- und Nachfiltrationskomponenten wie z.B. zusätzliche Eingangsfilter, als Kohle, Ultrafilter, oder auch als Sicherheitsfilter - Sterilfilter als Nachfilter möglich.

Es wird betont, dass die Erfindung nicht auf die beschriebenen und dargestellten Ausführungsformen beschränkt ist. Vielmehr sind alle offenbarten Merkmale der drei Ausführungsformen auch einzeln untereinander anders als oben beschrieben miteinander kombinierbar.

**Legende**

| | |
|---|---|
| 1. | Umkehrosmose (RO) |
| 2. | RO Filter |
| 3. | Druckrohr |
| 4. | RO Membran |
| 5. | Permeatsammelrohr |
| 6. | Anschluss-Wasserzufuhr-/Druckrohr |
| 7. | Anschluss Konzentrat |
| 8. | Permeatsammelrohr Aufnahme Reinigungszelle |
| 9. | Sekundärseite/ Permeattaschen |
| 10. | Primärraum/seite/ Wasserzuführungskanäle |
| 11. | Membran |
| 12. | Äußerer Membranmantel |
| 13. | Permeatsammelrohr Perforierungen |
| 14. | Permeattaschenverklebung |
| 15. | Druckrohrabschluss |
| 16. | Reinigungszelle Permeat |
| 17. | Reinigungszelle Wasserzuführung |
| 18. | Wassereingangsventil |
| 19. | Verbindungsleitung HD Gerät (Permeatleitung) |
| 20. | Leitfähigskeitszelle (LF-Zelle) |
| 21. | Hämodialysegerät (HD-Gerät) |
| 22. | HD-Spülventil |
| 23. | HD-Wassereingangsventil |
| 24. | HD-Wassereingangsbehälter mit Schwimmer- oder Niveausensoren |
| 25. | HD- Pumpe |
| 26. | HD-Spülleitung |
| 27. | Entgasungsdrossel |
| 28. | Entgasungsbeipassventil |
| 29. | HD-Heizung |
| 30. | Entgasungskammer |
| 31. | HD-Zirkulationsleitung |
| 32. | Konzentrat/Bikarbonatzuführung |
| 33. | Abfluss |
| 34. | Spülventil |
| 35. | Strömungswiderstand |
| 36. | Konzentratleitung |
| 37. | Pufferkammer |
| 38. | Zirkulationspumpe |
| 39. | Druckerhöhungspumpe |
| 40. | Elektroden |
| 41. | Membran |
| 42. | Kombinations Reinigungszelle |
| 43. | Äußere Elektroden |
| 44. | Innere Elektrode |
| 45. | Spulenwicklung |
| 46. | Ultraschallaufnahme |
| 47. | Aufnahme Dichtring |
| 48. | Isolierrohr |
| 49. | Zuführleitung |

## Patentansprüche

1. Kombination eines Einzelplatz-Umkehrosmosegerätes (RO-Gerät) (1) mit einem Hämodialysegerät (HD-Gerät) (21), wobei das RO-Gerät einen Filter mit einer Membran (11) aufweist, die einen Primärraum (10) von einem Sekundärraum (9) trennt, wobei in den Primärraum (10) eine Rohwasserzufuhrleitung (49) einmündet und der Sekundärraum (9) über eine eine Leitfähigkeits-Messeinrichtung (20) enthaltende Verbindungsleitung (19) mit einem Wassereingangsventil (23) des HD-Gerätes verbunden ist, und wobei der Konzentratausgang des Primärraums (10) über eine einen Strömungswiderstand (35) enthaltende Konzentratleitung (36) zu einem Abfluss (33) führt, wobei das Permeat bei Anforderung durch das HD-Gerät (21) entweder über das Wassereingangsventil (23) in das HD-Gerät oder über ein HD Spülventil (22) des HD-Gerätes zu einem Abfluss (33) fließt,
**dadurch gekennzeichnet,**
**dass** der Sekundärraum (9) ein Permeatsammelrohr (13) aufweist und
**dass** das Permeatsammelrohr (8) eine Reinigungskammer (16) enthält.

2. Kombination nach Anspruch 1,
**dadurch gekennzeichnet,**
**dass** die Reinigungskammer (16) in den von der Verbindungsleitung (19) abgewandten Endabschnitt des Permeatsammelrohres (13) eingebaut ist.

3. Kombination nach einem der Ansprüche 1 bis 2,
**dadurch gekennzeichnet,**
**dass** in die Rohwasserzufuhrleitung (49) eine Reinigungskammer (17) eingeschaltet ist.

4. Kombination nach einem der Ansprüche 1 bis 3,
**dadurch gekennzeichnet,**
**dass** die wenigstens eine Reinigungskammer (16,17) Oxidationsmittel zur Bekämpfung von Mikroorganismen erzeugt.

5. Kombination nach einem der Ansprüche 1 bis 4,
**dadurch gekennzeichnet,**
**dass** die wenigstens eine Reinigungskammer (16,17) Mittel zur Stabilisierung des im Wasser gelösten Kalks aufweist.

6. Kombination nach einem der Ansprüche 4 oder 5,
**dadurch gekennzeichnet,**
**dass** die wenigstens eine Reinigungskammer (16,17) mit elektrischen und/oder magnetischen und/oder elektromagnetischen und/oder elektrolytischen und/oder sonographischen Mitteln auf die durchfließende Flüssigkeit einwirkt.

7. Kombination nach einem der Ansprüche 1 bis 6,
**dadurch gekennzeichnet,**
**dass** eine mit einer Pumpe (38) versehene Rezirkulationsleitung die Konzentratleitung vor dem Strömungswiderstand (35) mit der Rohwasserzufuhrleitung vor der Reinigungskammer (17) verbindet.

8. Kombination nach einem der Ansprüche 1 bis 7,
**dadurch gekennzeichnet,**
**dass** die Verbindungsleitung (19) mit einer Permeat-Pufferkammer (37) versehen ist.

9. Kombination nach einem der Ansprüche 1 bis 8,
**dadurch gekennzeichnet,**
**dass** das Spülventil (34) so ausgebildet ist, dass es den Strömungswiderstand (35) öffnen kann.

10. Kombination nach einem der Ansprüche 1 bis 9,
**dadurch gekennzeichnet,**
**dass** die Membran (4) eine austauschbare Wegwerfmembran ist.

## Claims

1. Combination of a single plate reverse osmosis device (RO device) (1) with a haemodialysis device (HD device) (21), wherein the RO device has a filter with a membrane (11), which separates a primary space (10) from a secondary space (9), wherein an untreated water supply line (49) discharges into the primary space (10) and the secondary space (9) is connected via a connecting line (19), which includes a conductivity measuring device (20), to a water inlet valve (23) of the HD device and wherein the concentrate outlet of the primary space (10) leads via a concentrate line (36), which includes a flow resistance (35), to a discharge (33), wherein the permeate flows, when required by the HD device (21), either via the water inlet valve (23) into the HD device or via an HD flushing valve (22) of the HD device to a discharge (33), **characterised in that** the secondary space (9) has a permeate collecting pipe (5) and that the permeate collection pipe (5) includes a purificiation chamber (16).

2. Combination as claimed in Claim 1, **characterised in that** the purification chamber (16) is installed in the end section remote from the connecting line (19) of the permeate collecting pipe (5).

3. Combination as claimed in one of Claims 1 to 2, **characterised in that** a purification chamber (17) is connected into the untreated water supply line (49).

4. Combination as claimed in one of Claims 1 to 3, **characterised in that** the at least one purification chamber (16, 17) produces oxidising agent for the suppression of microorganisms.

5. Combination as claimed in one of Claims 1 to 4, **characterised in that** the at least one purification chamber (16, 17) has means for stabilising the lime dissolved in the water.

6. Combination as claimed in one of Claims 4 or 5, **characterised in that** the at least one purification chamber (16, 17) acts on the liquid flowing through it with electrical and/or magnetic and/or electromagnetic and/or electrolytic and/or sonographic means.

7. Combination as claimed in one of Claims 1 to 6, **characterised in that** a recirculation line provided with a pump (38) connects the concentrate line before the flow resistance (35) to the untreated water supply line before the purification chamber (17).

8. Combination as claimed in one of Claims 1 to 7, **characterised in that** the connecting line (19) is provided with a permeate buffer chamber (37).

9. Combination as claimed in one of Claims 1 to 8, **characterised in that** the flushing valve (34) is so constructed that it can open the flow resistance (35).

10. Combination as claimed in one of Claims 1 to 9, **characterised in that** the membrane (4) is a replaceable, disposable membrane.

## Revendications

1. Combinaison d'un appareil d'osmose inverse monoposte (appareil RO) (1) et d'un appareil d'hémodialyse (appareil HD) (21), dans laquelle l'appareil RO présente un filtre doté d'une membrane (11) qui sépare un espace primaire (10) d'un espace secondaire (9), dans laquelle dans l'espace primaire (10) débouche une conduite d'apport d'eau brute (49) et l'espace secondaire (9) est relié par une conduite de liaison (19) contenant un système de mesure de la conductibilité (20), à une soupape d'entrée d'eau (23) de l'appareil HD et
dans laquelle la sortie de concentré de l'espace primaire (10) conduit, par une conduite de concentré (36) contenant une résistance à l'écoulement (35), à une évacuation (33) dans laquelle le perméat s'écoule lors d'une sollicitation par l'appareil HD (21), soit par la soupape d'entrée d'eau (23) dans l'appareil HD, soit par une soupape de rinçage HD (22) de l'appareil HD, jusqu'à une évacuation (33),
**caractérisée en ce que** :
l'espace secondaire (9) présente un tube collecteur de perméat (13) et
le tube collecteur de perméat (8) contient une chambre de purification (16).

2. Combinaison selon la revendication 1,
**caractérisée en ce que** :
la chambre de purification (16) est montée dans la partie terminale détournée de la conduite de liaison (19) du tube collecteur de perméat (13).

3. Combinaison selon l'une des revendications 1 à 2,
**caractérisée en ce que** :
dans la conduite d'apport d'eau brute (49), une chambre de purification (17) est activée.

4. Combinaison selon l'une des revendications 1 à 3,
**caractérisée en ce que** :
l'au moins une chambre de purification (16, 17) produit des moyens d'oxydation pour lutter contre les microorganismes.

5. Combinaison selon l'une des revendications 1 à 4,
**caractérisée en ce que** :
l'au moins une chambre de purification (16, 17) présente des moyens de stabilisation du calcaire dissous dans l'eau.

6. Combinaison selon l'une des revendications 4 ou 5,
**caractérisée en ce que** :
l'au moins une chambre de purification (16, 17) agit avec des moyens électriques et/ou magnétiques et/ou électromagnétiques et/ou électrolytiques et/ou sonographiques, sur le liquide s'écoulant.

7. Combinaison selon l'une des revendications 1 à 6,
**caractérisée en ce que** :
une conduite de recirculation dotée d'une pompe (38) relie la conduite de concentré de la résistance d'écoulement (35) à la conduite d'apport d'eau brute de la chambre de purification (17).

8. Combinaison selon l'une des revendications 1 à 7,
**caractérisée en ce que** :
la conduite de liaison (19) est dotée d'une chambre de tampon de perméat (37).

9. Combinaison selon l'une des revendications 1 à 8,
**caractérisée en ce que** :
la soupape de rinçage (34) est conçue de telle sorte qu'elle peut ouvrir la résistance de l'écoulement (35).

10. Combinaison selon l'une des revendications 1 à 9,
**caractérisée en ce que** :
la membrane (4) est une membrane jetable échangeable.
